# EUROPEAN PATENT APPLICATION

(11) **EP 1 731 165 A1**
(43) Date of publication of application: **13.12.2006**
(21) Application number: 05012108.6
(22) Date of filing: 06.06.2005
(51) Int. Cl.: A61K 39/00, A61K 39/39, A61P 35/00, A61P 37/00, C12Q 1/68

(54) **Allogenic placenta-lysates/BCG-vaccine**

(71) Applicant: Corocleanu, Manole, 500025 Brasov (RO); Fruman, Radu, 500085 Brasov (RO)
(72) Inventor: Corocleanu, Manole, 500025 Brasov (RO); Fruman, Radu, 500085 Brasov (RO)

(57) **Abstract**

Placenta-Lysates preparation upon Filatov's method, as a vaccine-related product, consists of heat-shock protein and associated peptide complexes and take advantage on the fact that certain molecules on the surface of placenta cells are also found on cancer cells. Hsps are antigen carriers and enhancers of the immune response which cross-prime CTL responses, when attaching something that is definietly foreign known as adjuvant, e.g. BCG-Vaccine. By using the adjuvant as a decoy, the immune system may be tricked into attacking both the antigen/adjuvant complex (the composed vaccine) and the patient's nascent transformed cells. This vaccine that targets nonmutated proteins found on several different kinds of cancer cells, homologous with some placental proteins, and that would be expected in a subsets of patients with a defmed tumor histology, may be used to investigate a possible "universal cancer vaccine" that may cause an immune response against hyperplastic cells for commitment to differentiation or against nascent neoplastic cells that originate from any tissue, and that when administered to normal healthy individuals, has the potential to reduce the risk of cancer. On the other hand, extrinsic administration of Placenta-Lysates/BCG vaccine, as alternative to Hormonal Replacement Therapy, in patients with T-cell disorders, or cytokine dysregulation syndromes caused by inappropriate steroid modulation, by involvement in cytokine network interaction plays the role of an signaling supemode and causes changes in the pattern of cytokine expression that have bystander effects on other lymphocytes, resulting in T-cell regulation and Th1/Th2 balancing. Also, this results in activating the control mechanisms that maintain the correct constraints on the activity of autoreactive T cells in inflammatory non-organ-specific diseases, which include rheumatological disorders.

## Description

### Strategy for T-cell Immune Disorders-Susceptibility and Severity Testing and their Prevention and Therapy with Placenta-Lysates/ Bacillus Calmette-Guerin Vaccine.

The invention prospectively evaluated before the procedure becomes widely accepted as a valid method, concerns to use a combination of a biological pharmaceutical preparation of human placenta-tissue, upon Filatov's method (Suspensio Placentae pro injectionibus), as allogeneic whole-cell placenta vaccine-related product, Th2-inducing, admixed with BCG-Vaccine, a potent immunomodulator, Th1-inducing, the administration of which is intended to nonspecific stimulate regulatory T-cell mechanisms, fine-tune T-cell repertoire and Th1/Th2 balancing and thus, to result in prevention, amelioration and therapy of some T-cell disorders and cytokine dysregulation syndromes.

### Overexpression of normal proteins on hyperplastic and neoplastic epithelial cells makes them immunogenic.

Oncogenes are potent inducers of proliferation. Amplification and overexpression of some oncogenes play a significant role in human cancer. They are proteins derived from normal genes but are displayed at levels detectable by T cells on epithelial hyperplastic and neoplastic cells by overexpression to an abnormal high-density of specific peptide-MHC complexes that make them immunogenic (overexpression of normal "self antigens" considered as Tumor Associated Antigens). An example is HER-2/neu, (also known as c-Erb-2) V.T.DeVita Jr, S.Hellman, S.A.Rosenberg, "Cancer, Principles & Practice of Oncology", 6-th edition, Lippincott Williams & Wilkins, 2001), which is a receptor tyrosine kinase homologous to the epidermal growth factor receptor. This receptor is overexpressed in many carcinomas, including breast and ovarian cancers. MHC class I restricted, CD8-positive cytotoxic T lymphocytes have been found infiltrating solid tumors overexpressing HER-2/neu, but are not capable of destroying such tumors in vivo. Mielke S, Meden H, Kuhn W. (Med Hypotheses. 1998 May; 50(5):359-62) have shown that c-erb B-2-encoded oncoprotein p185 (HER-2/neu) is overexpressed on epithelial cells in the placenta and in several human carcinomas. It seems that autoreactive T cells develop against normal proteins derived from normal genes, only when overexpressed to an abnormally high density of specific peptide-MHC complexes displayed on the hyperplastic cells, that make them immunogenic. Oncogenes, as potent inducers of proliferation have been shown to sensitize cells to a wide variety of stimuli, including hypoxia and growth factor withdrawal. Conversely, overexpression of certain proteins could promote increased expression of stress-induced proteins, thus facilitating class I presentation. The ability of Heat shock proteins (Hsp) to chaperone other proteins, including peptides produced in stressed cells by ischemia and energy depletion, suggests that stress proteins (Heat shock proteins, Hsps) are antigen carriers (including of 'self antigens') and enhancers of the immune response.

### Placenta-proteins are found on different epithelial cancer cell.

During the last decades of the last century, H.Bohn and W.Winckler (Arch. Gynecol. Obstet. 248: 111-115. 1991; Arch. Gynecol. Obstet. 248-: 191-198, 1991) have isolated and immunochemically characterized, from hydrolyzed placenta-extracts and placenta membranes more than 30 new proteins, termed placenta proteins (PP) and placenta membrane proteins (MP). Of these, some are placenta specific, other ones being detected also, in other tissues, including cancerous, e.g. PP5, PP10, PP11, PP12, that were detected in trophoblast tumors and ovarian adenocarcinomas. The same proteins were detected also in breast carcinoma and digestive tumors. High serum levels of PP7 and PP8 were found in patients with hematological, digestive and respiratory malignant diseases; PP5, was found in lung, breast and gynecological cancers; PP11, was found in breast cancer (47%), ovary (67%), and in cancer of the stomach and of the testis (38%); PP10, was found in mucinous and serous ovarian cystadenocarcinomas, breast and endometrial cancers, and also in benign tumors of the breast, ovary and uterus.

### Allogeneic Placenta-Lysates-proteins crossreact with self- proteins expressed in epithelial hyperplastic and neoplastic tissues.

Our previous studies (Rev. Roum. Biochim. 18, 1, 7-13, 1981) have shown some exploitable qualities of a pharmaceutical allogeneic placenta-lysates, prepared upon Filatov's method (Suspensio Placentae pro injectionibus, Odesskyzavod Hinfarmapreparatov, Odessa, former URSS), for in vivo detection by skin testing (0,2 ml) of an existing immune response, characterized as a CD4+ T helper-cell-dependent delayed-type hypersensitivity (DTH) reaction, in obstetrical and gynecological patients with different clinical conditions, some specific to pregnancy and other with immune or inflammatory immune component characteristic for non-organ-specific diseases.

Analyzing the clinical conditions and the pathobiological substratum of patients that have shown cutaneous delayed-type hypersensitivity reaction (DTH-reaction) to Placenta-preparation, it was observed among immune and inflammatory immune diseases, also hyperplastic epithelial tissue proliferation e.g. endometrial hyperplasia, syncytiotrophoblast hyperplasia, but also different epithelial cancers, that qualifies Placenta-preparation upon Filatov'method as a vaccine-related product.

Thus, a positive selected T cells with a modest affinity for a self-peptide stand a good chance of recognizing the placental version of homologous peptides that have been found on hyperplastic and neoplastic cells. However, the resulting CD4 cell response did not involve the development of effector function. For stimulating expansion of CD8+ population in vivo via vaccine it is necessary to admix Placenta Lysates with an adjuvant e.g, BCG-Vaccine.

### Placenta accounts for the tolerance shown to the fetus and expresses oncofetal antigens.

It was hypothesized that placenta accounts for the tolerance shown to the fetus. This suggests that Th2 immunity characteristic of pregnancy, may be a natural response to trophoblast antigens, and would normally serve to downregulate Th1 response. The secretion of cytokines at the maternal-fetal interface might contribute to maternal tolerance of the fetus. (Janeway C. A. et all., "Immunobiology", 5-th edition, Garland Publishing 2001. Both hormonal modulated uterine epithelium and hormonal-secreting trophoblast, induce expression of Th2-type cytokines, including transforming growth factor-beta (TGF-beta), IL-4, and IL-10. This cytokine pattern tends to suppress Th1 responses. Thus, depending on hormonal modulation, activated macrophages release inhibitory molecules (e.g. TGF-beta, PGE2, IL-10) that suppress immune and inflammatory immune responses, including T-cell proliferation.

It has become clear recently that effector CD8+ T cells can, in addition of their familial cytolytic function, also respond to antigen by secreting cytokines typical of either Th1 or Th2 cells. CD8+ T cells that make Th2-associated cytokines are associated with regulatory and suppressor function, by producing cytokines such as IL-10 and TGF-beta. Thus, suppression of CD4 T cells by CD8 T cells, which have been observed in various situations, can be explained by their expression of different sets of cytokines.

Thus, immune response to placenta proteins, in an environment modulated by hormones, tends to settle into Th2-immunoregulatory-type in normal pregnancy or into Th1-effector response, when oncofetal proteins are overexpressed and become immunogenic e.g.sincytio-trophoblast hyperplasia, trophoblastic disease.

Oncofetal antigens are differentiation antigens expressed during fetal development but normally not expressed, or expressed at very low levels, in adult life. They are expressed at high levels by epithelial tumors ('self-antigens' or TAA).

Sarandakou A.et all. (Eur.J.Gynaec. Oncol. ISSN:o392-2936, Vol. XIX. N. 1, 1998. 73-77) have stressed that the involvement of some placental proteins (oncofetal) in both placental and malignant development (re-expression of embrionic antigens considered as Tumor Associated Antigens) supports the concept that these proteins may intervene in the control of maternal immune responses during pregnancy, in the manner of the host defence to carcinogenesis.

### Placenta-Lysates/BCG Vaccine cross-prime CTL and regulates T-cell responses.

It is suggested that in cryopreserved placenta tissue preparation (at 4°C for 6 days) upon Filatov's method, placenta cells stressed by energy depletion and lack of oxigen in the survival time after delivery, the bias towards Hsp production would result in greatly increased opportunities for Hsp binding to unregulated peptide fragments e.g. HER2/neu- encoded oncoprotein, oncofetal proteins (CEA, CA125, SCC and TPS), and to homologous placental proteins found by H.Bohn et all. on different kind of cancer cells, leading to an increase in potentially antigenic Hsp-peptide complexes, and recommending it as a vaccine-related product.

Heat shock proteins (Hsps) are a class of host-derived inflammatory mediators that perform the dual function of both chaperoning MHC class I-restricted epitopes into the cross-presentation pathway of DCs, as well as inducing the activation/maturation of these DCs to allow priming of cognate CD8⁺ T cell effector responses. The specific immunogenicity of Hsp is explained mechanistically by their ability to chaperone antigenic peptides derived from cellular proteins. These properties, and the ubiquitous nature of Hsps, make Hsp-peptide complexes attractive candidates for development of vaccines against both tumors and pathogens.

Members of HPS-family such as gp96 (Chuanliang Liu, Nigel Ewing, Melissa DeFilippo, James Zabrecky, Alem Truneh and Pramod Srivastava Antigenics Inc., 3 Forbes Road, Lexington, MA 02421, USA) and Hsp-40 (Biochim Biophys Acta. 1998 Mar 3; 1383(1):4-8). were detected in human placenta.

Investigations have shown that cell-associated proteins in the form of damaged cells and particulate antigens, after mechanically disrupted tissues for vaccine preparation, would gain more-efficient access to cross-presentation pathway. They are more effective at class I-restricted CTL priming than are simple soluble molecule.

It can be speculated that placenta particulate antigenic forms mimic a hormonal-modulated three-dimensional tissue, that tends to settle the immune response into Th2-type, but also express Hps-placenta protein-complexes-version of homologous proteins that are found on different kinds of epithelial cancer cells. By harnessing their immunogenicity when admixing with a potent adjuvant (BCG-Vaccine), may result in immune resistance to transformation, but also, by extrinsic manipulation of cytokine network, may results in regulation of disturbed immune responses, and thus in amelioration or limitation of immune or inflammatory immune responses. Thus, Hsps-placenta peptide complexes/cytokine inducing-based vaccine-related product could be used in preventive medicine to immunize healthy persons with a high risk of cancer to prevent the onset of tumors.

Young et all. ("Immunology Today", Vol. 19, No. 11, 502-505) have shown that the widespread expression of CD40 in normal epithelial cells and carcinoma cells suggests that this receptor has important additional influences beyond that of regulating immune responses, and that activation of these receptors might influence commitment to differentiation. The source of CD40 ligand (CD40L) at epithelial cell site could be delivered by activated T cell, particularly during immune responses. This demonstration suggests that the CD40-CD40L interaction is required for the generation of protective immunity and promotes differentiation of epithelial cells.

Altogether, these data suggest that Hsps derived from allogeneic placenta cells carrie placenta peptides homologous to tumor-derived peptides (TAA such as 'self antigens' and oncofetal antigens) that would be expected in a subsets of patients with a defined tumor histology. Because, these provide insufficient CD4+ cell help to support CD8 expansion it is necessary to harness their immunogenicity by admixing with a potent adjuvant (BCG-Vaccine). By intradermal injection, Hsps facilitate entry of exogeneous peptides or proteins e.g. Placenta-Lysates/BCG Vaccine into MHC class I pathway and can potentially mediate cross-presentation that occur via the key professional APCs, dendritic cells (DCs) and macrophages. CD4+ T-cell help acts through binding of CD40 on APCs causing upregulation of B7 and the induction of various cytokines important for T-cell-mediated immunity.

### Preparation of allogeneic whole-cell placenta tissue vaccine-related product upon Filatov'method.

Placentas harvested in sterile conditions from women in clinically and laboratory good conditions, are cryopreserved at 2°- 4°C for 6 days and then mechanically disrupted with a Polytron stainless steel high speed tissue homogenizer; 10 gr. of this paste is suspensed in 100 ml of distilled water and kept at room temperature for 1 h and then at 70°C for 1 h, followed by many cycles of filtration, until albumine concentration attain < 0,06 pro mille. Appropriate amount (1-2 ml) of placenta lysates is pouring down in vials followed by warming up at 70°C for 1 h and for consecutive 3 days.

All vaccine preparation will be proved sterile, mycoplasma free, nonpyrogenic, endotoxin free, and free of hepatitis virus and AIDS virus. Testing for sterility and general safety in test animals will be performed in commercial laboratories under current regulations.

### Components of the immune system interact in network and are modulated by hormones.

Parenchymal and stromal cells of various tissues communicate by means of cell-membrane contacts but also by means of soluble factors, especially cytokines. These signals released by the cells are short-range ones and control cell growth, differentiation, cell death, and effector functions, including the secretion of other cytokines, as is evident for immune cells. Cytokine-producing immune cells circulate from one tissue to another. The cell migration in part accounts for the overall interdependency of cytokines. The interdependency of these regulatory signals is displayed also at the level of individual tissues, because the release of a particular cytokine by effector cells depends on microenvironmental stimuli among which signals are mediated by other cytokines. Consequently, the complex physiologic homeostasis-taking place in the tissues is coordinated by the cytokine network, which is a set of interdependent regulatory cytokines and their corresponding receptors.

Imbalance in cytokine production or cytokine receptor expression and/or dysregulation of a cytokine process provides the basis for generating pathological disorders.

Pathological conditions, such as cancers, immune and inflammatory immune diseases, are associated with abnormal cytokine production, and the morbidity associated with many medical disorders is often directly a result of cytokine production. Because of the absence of negative feedback control occurring in some pathophysiologic situations, a given cytokine may flood and accumulate in the extracellular compartment of tissues or tumors thereby impairing the cytokine network homeostasis and contributing to local pathogenesis.

Suppressive influences can exist in any immune response. In some instances, suppression appears to be antigen-nonspecific. Possible mechanisms include an effect of cytokines that nonspecifically interfere with proliferation or differentiation of immune cells. On the other hand the immune response is promoted or limited by regulatory cells (by up-regulation of CTLA-4 on effector T cells) through their secreted cytokines and cell surface ligands, which as a group, form a network of overlapping activities that reduce the likelihood of general failure of an immune response, or of an immune response getting out of hand.

It is now apparent that such regulation is mediated by a whole spectrum of CD4 T cell subsets that do not only include Th2 cells but also non-Th2 cells such as Th3 cells (producing TGFβ), NK T cells (CD1d restricted), Tr1 cells (producing IL-10 but not IL-4) and CD4+ CD25+ T cells that require direct contact with their target cells (antigen presenting cells or effector cells).

Whatever their nature, effective products, will need regulate the balance of Th1/Th2 response, and promote the maturation of the regulatory T-cell circuits, by releasing cytokines that have bystander effects on other T cells and thus, suppress antigen-induced activation of other T lymphocytes, and activate the control mechanisms that maintain the correct constraints on the activity of autoreactive T cells. The result is a sensitive system of checks and balances that produces an immune response that is prompt, appropriate, effective and self-limiting.

In addition to these direct effects, the immune system is integrated with the nervous and endocrine system. Beside receptors for cytokines, many cells of the immune system express for hormones, including steroids, neurotransmitters, opioids, and neuropeptides.

The interplay between hormones and immune cells is not unidirectional. Cytokines, in particular IL-1, produced by macrophages and IL-6 produced by T cells, have been shown to have a role as bi-directional modulators of endocrine-immune communication. They act as circulating mediators of the acute phase response. Distant targets of these macrophage-derived cytokines include thermoregulatory centers in the CNS, muscle and fat stores, and the neuroendocrine system. Hormonal imbalance, considered to be basic to the development of multipatterned of target-tissue disorders, involves inadequate priming of Th-1 activity to an incorrect cytokine balance, as result of the interplay between these and individual cytokine profile. Thus, viewing the inappropriate or absence of steroid-modulation syndrome as a T-cell-mediated disease in which cytokine environment is key to the entire clinical phenotype, may serve as a working hypothesis, but one must consider also the impact of disease heterogeneity with respect to genetic background.

Clear evidence is emerging that the effects on bone mass is mediated by cytokines. Decreased estrogen levels result in increased secretion of IL-1, IL-6, and TNF-α by blood monocytes and bone marrow cells. These cytokines are potent stimulators of osteoclast recruitment and activity, and enhance bone breakdown. As the pattern of cytokines expressed by T lymphocytes is critical in determining, perpetuation and clinical expression of steroids imbalance, the manipulation of cytokine expression, by a vaccination strategy, offers a way of controlling it, but do not have the negative effects of estrogens on reproductive organs and on the cardiovascular system.

In conclusion, the complex physiologic homeostasis taking place in the tissues is coordinated by the cytokine network, which is a set of interdependent regulatory cytokines and their corresponding receptors. Identifying of intruders, or of functioning faults, as result among others also of inappropriate hormonal modulation, including steroids, immune network implements network-level recognition, by connecting information from local recognition agents by dynamical evaluation chains, as result of self-organizing and self-regulating characteristic of a complex system. However in the absence or excess of modulatory signals such as hormones, including steroids, the dysregulated cytokine overproduction may lead to a cytokine network homeostatic imbalance in the local concerned tissue.

A vaccination strategy that uses Placenta Lysates/BCG Vaccine in women, by generating broad T-cell cytokine responses and by incorporating the processed information into the cytokine regulatory network, is designed to reinforce the network by boosting its connectivity, and to trigger specific mechanisms that mount a defense response, but also to trigger self-organizing and self-regulating mechanisms.

### Individual cytokine profile.

The level of cytokines and magnitude of response is controlled at genetic level. It is likely that these genetic factors contribute to disease susceptibility and clinical severity, although environmental factors are also important. The inflammatory responses, therefore, is genetically programmed, with some individuals having a more vigorous response than others. However, a clear correlation of certain cytokine phenotype with disease activity does not exist. One must consider the impact of disease heterogeneity with respect to genetic background and clinical phenotypes. Cytokine profile can be used as a diagnostic tool for various disease models. Cytokines level provide us with methods of evaluating the systemic immune response.

By using simple genetic test, PCR ( Polymerase Chain Reaction) it is possible to determine whether someone is predisposed to make higher or lower amounts of certain cytokines. A person who is a higher producer of TNF-alfa, an inflammatory cytokine, and a lower producer of IL-10, an anti-inflammatory cytokine, is more susceptible to inflammatory conditions. Investigating individual cytokine profile it is possible to assess the susceptibility and severity of T-cell immune disorders, or cytokine dysregulation.

Cytokines play an important role in human health and disease. Monitoring their effects and detecting alterations in the complex balance of cytokines within a patient will undoubtedly become increasingly common in the clinical laboratory. ELISPOT-enzymatic test assay allows the detection of functional T cells according to the soluble mediators they release, i.e. cytokines. Cytokine Network Cytometry measuring soluble cytokines ("what"), cytokine-producing cells ("who"), surface receptors ("where".), and function (how) simultaneously are necessary to provide clinically useful information.

Thus, the proposal of individual genetic cytokine profile-test, as generic model for the long-range integration of genetic services into health care, would be to better inform interested people of their immune T-cell disorders-risk.

### Extrinsic immune modulation can affect T-cell disorders and cytokine dysregulation syndromes.

The Th1/Th2 paradigm became the key concept in immunoregulation. It has been demonstrated that Th1-Th2 balance is an important factor in immunoregulation and its imbalance becomes the cause of immune disorders. As the pattern of cytokine expressed by T lymphocytes is critical in determining, perpetuation and expression of inappropriate immune response, the manipulation of cytokine expression offers a way of controlling it. These involve manipulating the cytokine environment in which T-cell activation take place, or manipulating the way antigen is presented, as these factors have been observed to influence the differentiation and cytokine-secreting function of activated T cells.

Suppressive influences can exist in any immune response. In some instances, suppression appears to be antigen-nonspecific. Possible mechanisms include an effect of cytokines that nonspecifically interfere with proliferation or differentiation of immune cells. Whatever their nature, effective products, will need regulate the balance of Th1/Th2 response, and promote the maturation of the regulatory T-cell circuits, by releasing cytokines that have bystander effects on other T cells and thus, suppress antigen-induced activation of other T lymphocytes, and activate the control mechanisms that maintain the correct constraints on the activity of autoreactive T cells. It has become clear recently that effector CD8+ T cells can, in addition of their familial cytolytic function, also respond to antigen by secreting cytokines typical of either Th1 or Th2 cells. CD8+ T cells that make Th2-associated cytokines are associated with regulatory and suppressor function, by producing cytokines such as IL-10 and TGF-beta. Thus, suppression of CD4 T cells by CD8 T cells, which have been observed in various situations, can be explained by their expression of different sets of cytokines. (antiinflammatory cytokines).

The vaccination strategy that uses Placenta Lysates/BCG-Vaccine, by incorporating the processed information into the immune regulatory network, is designed to reinforce the network by boosting its connectivity, and to trigger specific mechanisms that mount a defense response, but also to trigger self-organizing and self-regulating mechanisms.

### Concluding remarks.

The fact that placenta proteins that have been found on different cancer cells are nonmutated has two important implications. First, expression of these proteins would be expected in a subset of patients with a defined tumor histology, thus a vaccine strategy targeting these antigens is possible. Second, the nonmutated nature of these proteins suggests that immunotherapies that target these antigens could elicit autoreactivity.

Placenta-Lysates preparation upon Filatov's method, as a vaccine-related product consists of heat-shock protein and associated peptide complexes and take advantage on the fact that certain molecules on the surface of placenta cells are also found on cancer cells. Hsps are antigen carriers and enhancers of the immune response which cross-prime CTL responses, when attaching something that is definietly foreign known as adjuvant, e.g. BCG-Vaccine. By using the adjuvant as a decoy, the immune system may be tricked into attacking both the antigen/adjuvant complex (the composed vaccine) and the patient's nascent transformed cells. This vaccine that targets nonmutated proteins found on several different kinds of cancer cells, homologous with some placental proteins, may be used to investigate a possible "universal cancer vaccine" that may cause an immune response against hyperplastic cells for commitment to differentiation and against nascent neoplastic cells that originate from any tissue, and that when administered to normal healthy individuals, has the potential to reduce the risk of cancer. A positive selected T cells with a modest affinity for a self-peptide stand a good chance of recognizing the placental version of homologous peptides derived from benign or malignant proliferation.

On the other hand, extrinsic administration of Placenta-Lysates/BCG vaccine in patients with susceptibility or severity for T-cell disorders, or cytokine dysregulation syndromes, by involvement in cytokine network interaction, playing the role of an signaling supernode, influences the differentiation and cytokine-secreting function of activated T cells and causes changes in the pattern of cytokine expression that have bystander effects on other lymphocytes, resulting in T-cell regulation and Th1/Th2 balancing, in patients with cytokine imbalance profile, caused by inappropriate steroid modulation. Also, this results in activating the control mechanisms that maintain the correct constraints on the activity of autoreactive T cells in inflammatory non-organ-specific diseases, which include rheumatological disorders, that characteristically involve the skin, kidney, joints and muscle, by releasing of appropriate cytokines, that have bystander effects on other T cells and suppresses antigen-induced activation of other T lymphocytes, downregulating proinflammatory cytokines (TNF-alfa) and upregulating anti-inflammatory cytokines (IL-10). Thus, by altering the balance between different subsets of responding T cells, and by shaping the cytokine milieu, causes changes in the pattern of cytokine expression that have bystander effects on lymphocytes with the presumed autoreactive receptors.

Thus, this combination of vaccines is not designed to protect against any specific disease, but rather to maintain the correct cytokine balance and the correct constraints on the activity of autoreactive T cells and thus, to result in prevention, amelioration, or therapy of some T-cell disorders or cytokine dysregulation syndromes.

This vaccine strategy design is prospectively evaluated, before the procedure becomes widely accepted.

Two mechanisms are proposed to fulfill this function: voluntary registries of procedure undertaken and prospective multicenter randomized trials.

Also, the proposal of individual genetic cytokine profile-test, as generic model for the long-range integration of genetic services into health care, would be to better inform interested people of their immune T-cell disorders-risks.

### References.

1. Corocleanu M., Rev. Roum Biochim. 18, 1, 7-13, 1981.
2. H.Bohn and W.Winckler, Arch. Gynecol. Obstet, 248: 111-115, 1991; Arch. Gynecol. Obstet. 248-: 191-198, 1991.
3. C.A. Janeway, P.Travers, M.Walport, M. Shlomchik, "Immunobiology", 5-th edition, Garland Publishing 2001.
4. Mielke S, Meden H, Kuhn W. Med Hypotheses. 1998 May;50(5):359-62.
5. Roitt, Brostoff, Male, "Immunology" 6-th edition, Mosby 2001.
6. Pacifici, Proc.Natl. Acad. Sci.USA, august 4, 2004.
7. Sarandakou A. et all. Eur.JGynaec. Oncol. ISSN:o392-2936, Vol. XIX. N. 1, 1998. 7-77.
8. V.T.DeVita Jr, S.Hellman, S.A.Rosenberg, "Cancer, Principles & Practice of Oncology", 6-th edition, Lippincott Williams & Wilkins, 2001.
9. Young et all."Immunology Today", Vol. 19, No. 11, 502-505.

## Claims

1. A combination of vaccines, composed of a pharmaceutical whole-cell Placenta-Lysates, prepared upon Filatov's method, admixed with an adjuvant, e.g.bacillus Calmette Guerin (BCG)- Vaccine when administered by intradermal route, at a ratio of 0,1:0,1 ml., in patients with T-cell disorders or cytokine dysregulation syndromes, is designed to fine-tune expression of the T-cell repertoire and to promote the maturation of regulatory T-cell circuits and Th1/Th2 balancing, by extrinsically regulating the balance between different subsets of responding T cells such that helpful responses are promoted and damaging responses are suppressed.
This combination of vaccines is not designed to protect against any specific disease, but rather to maintain the correct cytokine balance and the correct constraints on the activity of autoreactive T cells and thus, to result in prevention, amelioration, or therapy of some T-cell disorders or cytokine dysregulation syndromes.

2. A combination of vaccines as claimed in claim 1, to activate the control mechanisms that maintain the correct constraints on the activity of autoreactive T cells in inflammatory non-organ-specific diseases, which include rheumatological disorders, that characteristically involve the skin, kidney, joints and muscle, by releasing of appropriate cytokines, that have bystander ettects on other T cells and suppresses antigen-induced activation of other T lymphocytes, downregulating proinflammatory cytokines (TNF-alfa) and upregulating anti-inflammatory cytokines (IL-10). Thus, by altering the balance between different subsets of responding T cells, and by shaping the cytokine milieu, causes changes in the pattern of cytokine expression that have bystander effects on lymphocytes with the presumed autoreactive receptors.

3. A combination of vaccines as claimed in claim 1, as alternative therapy to Hormonal Replacement Therapy (HRT), to elicit the release of critical cytokines at the vaccination site, and thus restoring and maintaining the correct cytokine balance in any site of the cytokine network in which a imbalance has appeared as result of inappropriate or absence of sex-steroids modulation.

4. A combination of vaccines as claimed in claim 1, to prevent tumor development, by boosting the potential capabilities of CD4+ T cells that react against oncoproteins and oncofetal proteins when are overexpressed, or against placenta-proteins homologous with proteins that have been found on different epithelial hyperplastic and neoplastic cells, and thus activating a potent cytolytic T lymphocyte (CTL) response, that may causes crossprotection in normal healthy individuals against some histologic variables of epithelial-cell hyperplasia of potential capabilities to progress towards transformation.
This "universal cancer vaccine" may cause an immune response against hyperplastic and neoplastic cells that originate from " transit" cells in epithelial tissues, and that when administered to normal healthy individuals, has the potential to prevent the onset of tumors and thus to reduce the risk of some kinds of epithelial cancers.

5. A combination of vaccines as claimed in claim 1, but replacing allogeneic Placenta Lysates with autogenous placenta in order to supply new-born with correct inputs for priming T helper 1 (Th1) activity, leading to a correct cytokine balance and for fine-tuning T-cell repertoire, thus reducing the incidence of autochthonous cancer.

6. The proposal of individual genetic cytokine profile-test, as generic model for the long-range integration of genetic services into health care, would be to better inform interested people of their immune T-cell disorders-risks. A patient needs to be thoroughly tested to identify not only the immediate problems but also their reaction to a cytokine-based vaccine strategy design, and only after a complete assessment of the patient diseases is performed should a multi-pronged cytokine therapy administered.

## Amended claims

### Amended claims in accordance with Rule 86(2) EPC.

**1.** Use a combination of vaccines, consisting essentially of a pharmaceutical allogeneic human Placenta-Lysates, prepared upon Filatov's method, as a vaccine-related product, admixed and intradermal co-injected with a potent adjuvant, e.g. BCG-Vaccine at a 1:1 ratio, for becoming an immunogenic and immunoregulatory drug product, the administration of which in normal healthy individuals has the potential to reduce the risk of cancer, by fine-tuning expression of T-cell repertoire and by enhancing immunogenicity of Heat shock proteins (Hsps)/placenta proteins (e.g. differentiation antigens, oncoproteins, oncofetal proteins) complexes-version of homologous nonmutated proteins that are found on different kinds of epithelial cancer cells, to result in potential cytolytic T lymphocyte (CT) response against nascent epithelial cancer of all types and from all origins.

**2.** Use of combination of vaccines of claim 1, for becoming a drug product for treating inflammation/associated immune disorders by activating the control mechanisms that maintain the correct constraints on the activity of autoreactive T cells in patients with inflammatory non-organ-specific diseases, which include rheumatological disorders, that characteristically involve the skin, kidney, joints and muscle, through shaping the cytokine milieu and by releasing of appropriate cytokines, that have bystander effects on other T cells and suppresses antigen-induced activation of other T lymphocytes, downregulating proinflammatory cytokines (TNF-alfa) and upregulating anti-inflammatory cytokines (IL-10).

**3.** Use of combination of vaccines of claim 1 for becoming a drug product, as alternative to Hormonal Replacement Therapy (HRT), for treating patients with cytokine dysregulation syndromes as result of inappropriate or absence of sex-steroid modulation of cytokine network, e.g. osteoporosis, woman's climacteric syndrome, by altering the balance between different subsets of responding T cells, and by shaping the cytokine milieu, causing changes in the pattern of cytokine expression and thus, restoring and maintaining the correct cytokine balance.
